# FASCICULE DE BREVET EUROPEEN

(11) **EP 1 466 583 B1**
(45) Date de publication et mention de la délivrance du brevet: **03.09.2008**
(21) Numéro de dépôt: 04290713.9
(22) Date de dépôt: 16.03.2004
(51) Int. Cl.: A61K 8/88, A61Q 5/12

(54) **Dérivé de N-alpha et N-epsilon-polylysine et polyornithine à fonction thiol et leur utilisation en cosmétique**
Thiolderivate von N-alpha und N-epsilon-Polylysine und Polyrnithine und deren Verwendung in der Kosmetik
Derivatives of N-alpha and N-epsilon-Polylysine and Polyornithine with a thiol function and their use in cosmetics

(30) Priorité: 11.04.2003 FR 0304575
(43) Date de publication de la demande: 13.10.2004
(73) Titulaire: L'ORÉAL, 75008 Paris (FR)
(72) Inventeur: Philippe, Michel, 91320 Wissous (FR); Andrean, Hervé, 75014 Paris (FR)
(74) Mandataire: Bourdeau, Françoise

(56) Documents cités:
- WO-A-99/37279
- US-A- 5 434 060
- MAHMOOD A ET AL: "A New Approach to Labeling Cells with Technetium-99m, Part I. Preparation of Modified Polylysine and In Vitro Cell Labeling" NUCLEAR MEDICINE AND BIOLOGY, ELSEVIER SCIENCE PUBLISHERS, NEW YORK, NY, US, vol. 23, no. 1, 1996, pages 79-85, XP004051695 ISSN: 0969-8051
- DATABASE CA [en ligne] CHEMICAL ABSTRACTS SERVICE, COLUMBUS, OHIO, US; PROSERPIO, G. ET AL: "Cosmetic properties and applications of amino-lysine thio ethers" retrieved from STN Database accession no. 84:65170 XP002267105 & RIVISTA ITALIANA ESSENZE, PROFUMI, PIANTE OFFICINALI, AROMI, SAPONI, COSMETICI, AEROSOL (1975), 57(3), 119-27 ,

## Description

L'invention a pour objet l'utilisation en cosmétique de dérivés poly-N-α et N-ε-lysines et ornithine à fonction thiol. Elle vise également de nouveaux dérivés poly N-α et N-ε-lysines et ornithine à fonction thiol ainsi que des compositions cosmétiques contenant ces nouveaux composés.

Il est connu des homo-oligomères et homo-polymères à base de polylysine (N-α et N-ε) en cosmétique, notamment dans le cadre du soin de la fibre (JP2002293719) et de leur activité antiseptique (JP2001328920).

Cependant ces composés présentent des problèmes d'écotoxicité importants qui limitent fortement leur potentiel d'application. Il est également nécessaire d'améliorer la rémanence de ces polymères cationiques sur la fibre.

Le terme "matière kératinique" englobe la peau, les ongles et les fibres kératiniques. On entend par *"fibres kératiniques",* les cheveux, les cils, les sourcils, les poils et notamment les cheveux. La présente invention vise en particulier les fibres kératiniques.

Au sens de la présente invention, on entend par *"dépôt rémanent sur les fibres kératiniques",* un revêtement continu ou non formé sur chaque fibre restant présent après cinq shampooings consécutifs.

Dans la définition indiquée ci-avant du terme *"dépôt rémanent sur les fibres kératiniques",* le revêtement peut représenter, d'un point de vue chimique, les polylysines selon l'invention, seules ou encore les polylysines en mélange avec d'autres composés cosmétiquement actifs.

Au sens de la présente invention, on entend par *« gainage* », une enveloppe formée à la surface de chaque fibre, notamment chaque cheveu, après séchage de la composition cosmétique.

On utilise l'abréviation *''poly N-α- et N-ε- lysine et ornithine",* pour -dénommer ensemble les dérivés poly-N-α- lysine, poly-N-α-ornithine, poly-N-ε-lysine et poly-N-ε-omithine. On utilise l'abréviation *''poly- N-ε-lysine et ornithine",* pour dénommer ensemble les dérivés poly-N-ε-lysine et poly-N-ε-omithine. La configuration de la lysine et de l'ornithine peut être L ou D ou encore un mélange des deux.

Le problème posé par la présente invention est de fournir des polylysines formant un dépôt rémanent sur les matières kératiniques, et notamment sur les cheveux et apportant, en outre, de bonnes propriétés cosmétiques aux matières kératiniques, et notamment aux cheveux, comme de la douceur, du lissage et un meilleur démêlage.

Pour résoudre ce problème, l'invention propose l'utilisation des poly N-α- et N-ε- lysine et ornithine de formule I, à fonction thiol, pour former un dépôt rémanent sur les matières kératiniques, et notamment sur les fibres kératiniques: 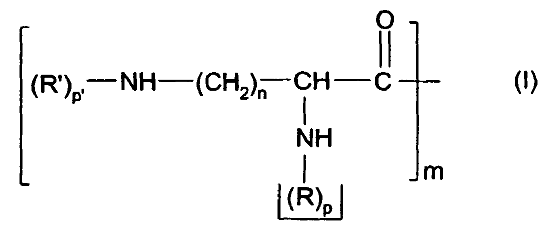 dans laquelle:
P différent de P' et P, P' = 0 ou 1
n=3ou4
Si P' = 0, alors le NH voisin est engagé dans une polymérisation N-ε ;
Si P = 0, alors le NH voisin est engagé dans une polymérisation N-α;
Si P ou P' = 1, alors R ou R' = A-SH, avec

- A = chaîne hydrocarbonée en C-1 à C-30, saturée ou non, linéaire ou ramifiée pouvant être interrompue par un ou plusieurs hétéro-atomes ou fonctions tels 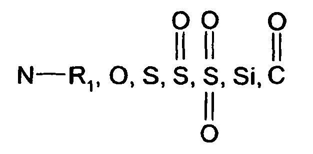 ou/et par un ou plusieurs cycles aromatiques ou non aromatiques (à 5, 6 ou 7 chaînons) et pouvant être substituée par un ou plusieurs groupements : COOH, OH, NH₂, alkyl (C-1 à C-8) amino, acyl (C-1 à C-8) amino, acyl (C-1 à C-8) oxy, alkyl (C-1 à C-8) oxycarbonylamino, alkyl (C-1 à C-8) amino-carbonyloxy, halogéno, alkyl (C-1 à C-8) aminocarbonyl,
   sachant que R ou R' peuvent, en partie seulement, également représenter H, ou/et 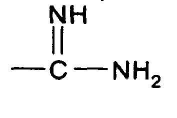 et ses sels ou/et 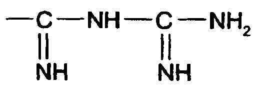 et ses sels.
- R₁ = H, alkyl (C-1 à C-8), acyl (C-1 à C-8), alkyl (C-1 à C-8) oxycarbonyl, alkyl (C-1 à C-8) amino-carbonyl, halogéno.
- A peut être également représenté par un cycle aromatique ou non aromatique à 5, 6 ou 7 chaînons, éventuellement substitués par les mêmes groupements déjà listés pour la chaîne aliphatique.
- m = 3 à 10000.

De préférence, le taux de greffage de fonction thiol sera supérieur ou égal à 1 %.

Avantageusement, les poly N-α- et N-ε- lysine et omithine répondant à la formule I présentent: 5<m<1000.

Le *"taux théorique de greffage de fonction thiol"* représente le pourcentage théorique en unité lysine ou ornithine porteuse de la fonction thiol dans le composé de formule I.

Avantageusement, on utilise des poly N-α- et N-ε- lysine et ornithine de formule I, à fonction thiol, en association ou non avec un actif cosmétique conventionnel pour former un dépôt rémanent sur les matières kératiniques.

Le caractère fortement cationique des composés de formule générale (I) conduit, une fois ceux-ci déposés sur la fibre, à une bonne adhésion d'actifs cosmétiques conventionnels anioniques, comme des colorants, conditionneurs, hydratants, émollients ou filtres solaires. Ces actifs possèdent pourtant, lorsqu'ils sont utilisés seuls, une très faible affinité pour la matière kératinique.

Un autre grand avantage des polylysines de l'invention est de posséder une réactivité covalente, par exemple avec les cheveux « via » la formation de liaisons disulfures. Ce point renforce encore l'intérêt pour l'obtention d'une rémanence importante sur la fibre, comparativement à des polymères cationiques non fonctionnalisés par des fonctions thiol

Plus avantageusement, on utilise des poly N-α- et N-ε- lysine et omithine de formule I, à fonction thiol, en association avec au moins un actif cosmétique conventionnel anionique, comme des colorants, conditionneurs, hydratants, émollients ou filtres solaires, pour former un dépôt rémanent sur les matières kératiniques.

Un autre objet de l'invention concerne de nouveaux dérivés de poly- N-ε et poly N-α-lysine et omithine à fonction thiol répondant à la formule II ci-après: 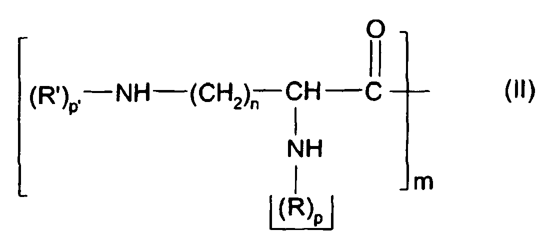 P différent de P' et P, P' = 0 ou 1
n = 3 ou 4
Quand
- P' = 0, le NH voisin est engagé dans une polymérisation N-ε ; quand P = 0, le NH voisin est engagé dans une polymérisation N-α.
Quand
- P ou P' = 1, alors R ou R' = A'-SH
- 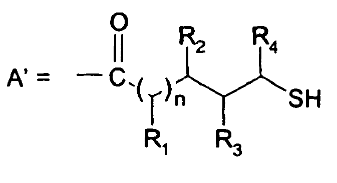
- R₁, R₂, R₃, R₄ = H, COOH, OH, NH₂, alkyl (C-1 à C-8) amino, acyl (C-1 à C-8) amino, acyl (C-1 à C-8) oxy, alkyl (C-1 à C-8) oxycarbonylamino, alkyl (C-1 à C-8) amino-carbonyloxy, halogéno, alkyl (C-1 à C-8) aminocarbonyl.
   sachant que R ou R' peuvent, en partie seulement, également représenter H, ou/et 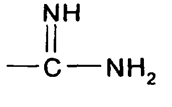 et ses sels ou/et 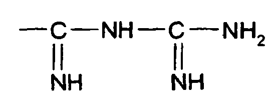 et ses sels.
- m = 3 à 10000.

De préférence, le taux de greffage de fonction thiol sera supérieur ou égal à 1 %.

L'invention concerne également des compositions cosmétiques comprenant, dans un milieu cosmétiquement acceptable, au moins un dérivé de poly-N-ε et poly N-α-lysine et omithine à fonction thiol de formule II.

Dans les compositions conformes à l'invention, le ou les dérivés de poly-N-ε et poly N-α-lysine et omithine à fonction thiol de formule Il sont, de préférence, présents à des concentrations comprises entre 0,05 et 30 % en poids, plus préférentiellement comprises entre 0,1 et 15 % en poids, et plus préférentiellement entre 0,25 et 10 % en poids par rapport au poids total de la composition.

Conformément à l'invention, la composition contient avantageusement, en outre, des additifs cosmétiques conventionnels choisis parmi les polymères fixants, les épaississants, les tensioactifs anioniques, non ioniques, cationiques ou amphotères, les parfums, les conservateurs, les protéines, les vitamines non réactives, les provitamines non réactives, les polymères non fixants anioniques, non ioniques, cationiques ou amphotères, les huiles minérales, végétales ou synthétiques, les silicones volatiles ou non, linéaires ou cycliques, modifiées ou non, des agents régulateurs de pH, des oxydants, des réducteurs, des catalyseurs et tout autre additif classiquement utilisé dans les compositions cosmétiques destinées à être appliquées sur les matières kératiniques, notamment sur les cheveux.

Le milieu cosmétiquement acceptable est, de préférence, constitué par de l'eau et/ou un ou plusieurs solvants cosmétiquement acceptables tels que des alcools, les esters, les cétones ou les silicones volatiles cycliques ou des mélanges eau-solvant(s), ces solvants étant de préférence des alcools en C₁-C₄.

Lorsque la composition selon l'invention est conditionnée dans un dispositif aérosol, elle comprend au moins un agent propulseur, qui peut être choisi parmi les hydrocarbures volatils tels que le n-butane, le propane, l'isobutane, le pentane, les hydrocarbures halogénés et leurs mélanges. On peut également utiliser en tant qu'agent propulseur le gaz carbonique, le protoxyde d'azote, le diméthyléther (DME), l'azote, l'air comprimé. On peut aussi utiliser des mélanges de propulseurs. De préférence, on utilise le diméthyl éther.

Avantageusement, l'agent propulseur est présent à une concentration comprise entre 5 et 90 % en poids par rapport au poids total de la composition dans le dispositif aérosol et, plus particulièrement, à une concentration comprise entre 10 et 60 %.

Les compositions conformes à l'invention peuvent être appliquées notamment sur des cheveux secs ou humides.

Les dérivés de poly-N-ε et poly N-α-lysine et omithine à fonction thiol de formule II sont préparés, de façon générale, par réaction sous atmosphère inerte, par exemple, de poly-N-ε-lysine fournie notamment par la compagnie Chisso ou de poly-N-ε-lysine à fonction guanidine ou biguanide avec une thiolactone comme la N-acétyl-homocystéinethiolactone fournie notamment par la société Fluka. La durée de la réaction peut notamment varier entre 30 minutes et 24 heures. Préférentiellement, la température de la réaction est comprise entre 0 et 80°C. Les polylysines peuvent éventuellement être partiellement salifiées.

La composition de l'invention peut être à usage cosmétique ou pharmaceutique. Préférentiellement, la composition de l'invention est à usage cosmétique. Aussi, la composition doit contenir un milieu physiologiquement acceptable non toxique et susceptible d'être appliqué sur la peau, les phanères ou les lèvres d'êtres humains. Par "cosmétique", on entend au sens de l'invention une composition d'aspect, d'odeur et de toucher agréables.

En particulier, la composition à application sur le cuir chevelu ou les cheveux peut se présenter sous forme d'une lotion de soin capillaire, par exemple d'application journalière ou bi-hebdomadaire, d'un shampooing ou d'un après-shampooing capillaire, en particulier d'application bi-hebdomadaire ou hebdomadaire, d'un savon liquide ou solide de nettoyage du cuir chevelu d'application journalière, d'un produit de mise en forme de la coiffure (laque, produit pour mise en pli, gel coiffant), d'un masque traitant, d'une crème ou d'un gel moussant de nettoyage des cheveux. Elle peut encore se présenter sous forme de teinture ou de mascara capillaire à appliquer au pinceau ou au peigne.

Un autre objet de l'invention concerne l'utilisation de dérivés de poly-N-ε et poly N-α-lysine et omithine à fonction thiol répondant à la formule II en cosmétique. Il est notamment appliqué sur la peau, les ongles ou les fibres kératiniques.

Par exemple, le composé de formule II peut être présent dans un vernis à ongles ou un produit de soin de la peau, un produit de maquillage ou un produit anti-âge, tout comme dans un produit capillaire de mise en forme, de soin des cheveux ou de coloration des cheveux.

L'invention va être plus complètement illustrée à l'aide des exemples non limitatifs suivants.

### EXEMPLES

On réalise trois polylysines de formule Il et de configuration L conformes à l'invention, appelées Ex.1, Ex.2, et Ex.3 .
Ex. 1 Structure (II)
   n=4,p'=0,p=1, 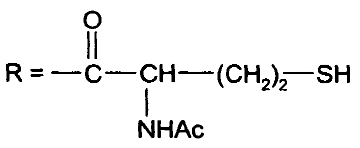 avec un taux de greffage de 10 %(reste R = H), m moyen = 37.
Ex. 2 Structure (II)
   n=4,p'=0,p=1, 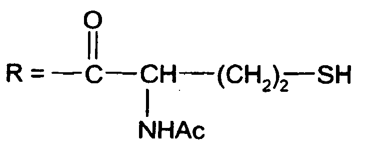 avec un taux de greffage de 30 % (reste R = H), m moyen = 37.
Ex. 3 : Structure (II)
   n=4,p'=0, p=1, 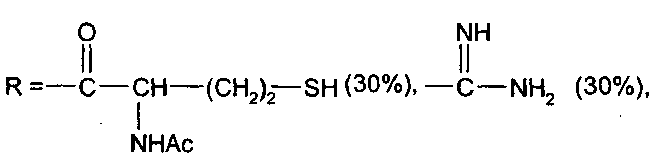 reste R=H La structure étant partiellement salifiée (HCI)

### Synthèse de Ex. 1

On charge 25 g de solution aqueuse de polylysine Chisso ( à 25 % de M.A. masse moyenne 4700) et 0,782 g de N-acétyl-homocystéinethiolactone.

### Mode opératoire

On dégaze la solution de polylysine par bullage d'argon.
A température ambiante et sous argon, on ajoute par petites fractions, en attendant la dissolution du réactif entre chaque addition, la N-acétyl-homocystéinethiolactone à la solution de polylysine sous agitation. On laisse sous agitation sous argon, pendant 24 heures, en suivant en CCM CH₂Cl₂/MeOH/NH₃ (15/4/1), la disparition du réactif et l'apparition de la fonction thiol sur le polymère qui ne migre pas, par révélation par le nitroprussiate de sodium.

L'analyse RMN ¹³C est conforme à la structure attendue.

Ex. 2 a été préparé selon le même mode opératoire que l'exemple 1 en augmentant la quantité de N-acétyl-homocystéinethiolactone (2,35 g).

On observe une rémanence à 10 shampooings pour Ex. 1 et Ex.2.

Ex. 3 : (Polylysine aminée, guanylée à 30 % et thiolée à 30 %).
12 g de solution de polylysine sont partiellement salifiés par 0,5 ml d'acide chlorhydrique concentré ; après addition de 1,04 g de 1-H-pyrazole carboxamidine mono-hydrochloride, le milieu réactionnel est chauffé 2h00 à 40°C, sous agitation. (Suivi de la réaction par RMN ¹H) .
Après extraction du pyrazole à l'éther éthylique, et dégazage sous argon, on ajoute à la solution aqueuse, toujours sous argon, 1,13 g de N-acétyl-homocystéine thiolactone ; on laisse agiter, sous argon, 16h00 à 40°C (suivi de la réaction par RMN et CCM), puis le pH de la solution est amené à 5, par ajout d'HCl concentré.

L'analyse RMN proton et ¹³C est conforme à la structure chlorhydrate attendue.

## Revendications

1. Utilisation de poly N-α- et N-ε- lysine et ornithine de formule I, à fonction thiol, pour former un dépôt rémanent sur les matières kératiniques, et notamment sur les fibres kératiniques: 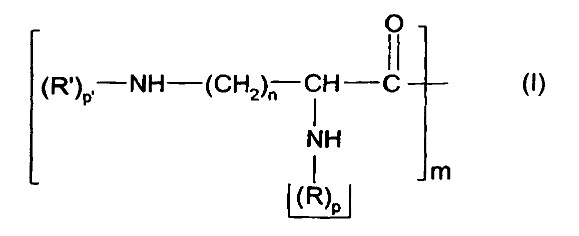 dans laquelle:
P différent de P' et P, P' = 0 ou 1
n = 3 ou 4
Si P' = 0, alors le NH voisin est engagé dans une polymérisation N-ε ;
Si P = 0, alors le NH voisin est engagé dans une polymérisation N-α;
Si P ou P' = 1, alors R ou R' = A-SH, avec
• A = chaîne hydrocarbonée en C-1 à C-30, saturée ou non, linéaire ou ramifiée pouvant être interrompue par un ou plusieurs hétéro-atomes ou fonctions tels 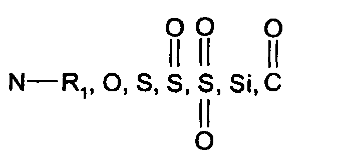 ou/et par un ou plusieurs cycles aromatiques ou non aromatiques (à 5, 6 ou 7 chaînons) et pouvant être substituée par un ou plusieurs groupements : COOH, OH, NH₂, alkyl (C-1 à C-8) amino, acyl (C-1 à C-8) amino, acyl (C-1 à C-8) oxy, alkyl (C-1 à C-8) oxycarbonylamino, alkyl (C-1 à C-8) amino-carbonyloxy, halogéno, alkyl (C-1 à C-8) aminocarbonyl,
sachant que R ou R' peuvent, en partie seulement, également représenter
H, ou/et 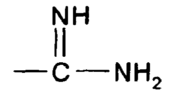 et ses sels ou/et 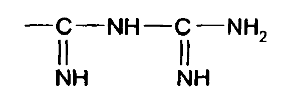 et ses sels.
• R₁ = H, alkyl (C-1 à C-8), acyl (C-1 à C-8), alkyl (C-1 à C-8) oxycarbonyl, alkyl (C-1 à C-8) amino-carbonyl, halogéno.
• A peut être également représenté par un cycle aromatique ou non aromatique à 5, 6 ou 7 chaînons, éventuellement substitués par les mêmes groupements déjà listés pour la chaîne aliphatique.
• m = 3 à 10000.

2. Utilisation selon la revendication 1, les poly N-α- et N-ε- lysine et ornithine répondant à la formule **I** dans laquelle: 5<m<1000.

3. Utilisation selon l'une quelconque des revendications précédentes, les poly N-α- et N-ε- lysine et ornithine présentant un taux de greffage de fonction thiol supérieur ou égal à 1%.

4. Utilisation selon l'une quelconque des revendications précédentes, les poly N-α- et N-ε- lysine et ornithine de formule I, à fonction thiol, étant en association avec au moins un actif cosmétique conventionnel pour former un dépôt rémanent sur les matières kératiniques.

5. Utilisation selon la revendication 4, l'actif cosmétique conventionnel étant un actif cosmétique conventionnel anionique, comme des colorants, conditionneurs, hydratants, émollients ou filtres solaires.

6. Dérivés de poly- N- ε et poly N-α-lysine et ornithine à fonction thiol répondant à la formule II ci-après: 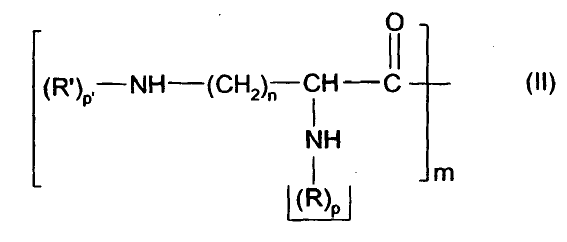
P différent de P' et P, P' = 0 ou 1
n = 3 ou 4
Quand
• P' = 0, le NH voisin est engagé dans une polymérisation N-ε ; quand P = 0, le NH voisin est engagé dans une polymérisation N-α.
Quand
• P ou P' = 1, alors R ou R' = A'-SH
• 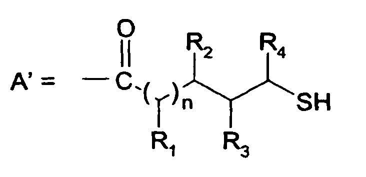
• R₁, R₂, R₃, R₄ = H, COOH, OH, NH₂, alkyl (C-1 à C-8) amino, acyl (C-1 à C-8) amino, acyl (C-1 à C-8) oxy, alkyl (C-1 à C-8) oxycarbonylamino, alkyl (C-1 à C-8) amino-carbonyloxy, halogéno, alkyl (C-1 à C-8) aminocarbonyl.
sachant que R ou R' peuvent, en partie seulement, également représenter
H, ou/et 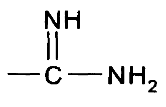 et ses sels ou/et 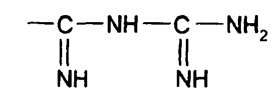 et ses sels.
• m = 3 à 10000.

7. Dérivés de poly- N- ε et poly N-α-lysine et ornithine à fonction thiol selon la revendication 6, **caractérisés par le fait que** le taux de greffage de fonction thiol est supérieur ou égal à 1 %.

8. Composition cosmétique comprenant, dans un milieu cosmétiquement acceptable, au moins un dérivé de poly- N- ε et poly N-α-lysine et ornithine à fonction thiol répondant à la formule II ci-après: 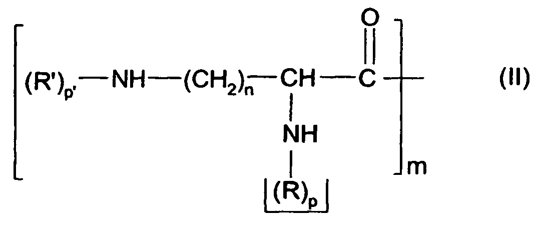
P différent de P' et P, P' = 0 ou 1
n = 3 ou 4
Quand
• P' = 0, le NH voisin est engagé dans une polymérisation N-ε ; quand P = 0, le NH voisin est engagé dans une polymérisation N-α.
Quand
• P ou P' = 1, alors R ou R' = A'-SH
• A'= 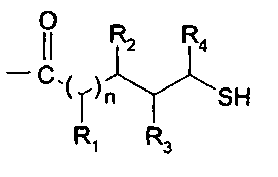
• R₁, R₂, R₃, R₄ = H, COOH, OH, NH₂, alkyl (C-1 à C-8) amino, acyl (C-1 à C-8) amino, acyl (C-1 à C-8) oxy, alkyl (C-1 à C-8) oxycarbonylamino, alkyl (C-1 à C-8) amino-carbonyloxy, halogéno, alkyl (C-1 à C-8) aminocarbonyl.
sachant que R ou R' peuvent, en partie seulement, également représenter
H, ou/et 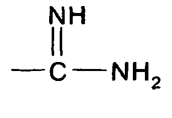 et ses sels ou/et 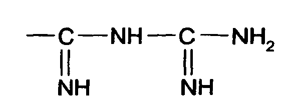 et ses sels.
• m = 3 à 10000.

9. Composition selon la revendication 8, **caractérisée par le fait que**, le ou les dérivés de poly- N- ε et poly N-α-lysine et ornithine à fonction thiol de formule II sont présentes à des concentrations comprises entre 0,05 et 30 % en poids, plus préférentiellement comprises entre 0,1 et 15 % en poids, et plus préférentiellement entre 0,25 et 10 % en poids par rapport au poids total de la composition.

10. Composition selon la revendication 8 ou 9, **caractérisée par le fait que** le milieu cosmétiquement acceptable est constitué par de l'eau et/ou un ou plusieurs solvants cosmétiquement acceptables tels que des alcools, les esters, les cétones ou les silicones volatiles cycliques ou des mélanges eau-solvant(s), ces solvants étant de préférence des alcools en C₁-C₄.

11. Utilisation en cosmétique de dérivés de poly- N- ε et poly N-α-lysine et ornithine à fonction thiol répondant à la formule II ci-après: 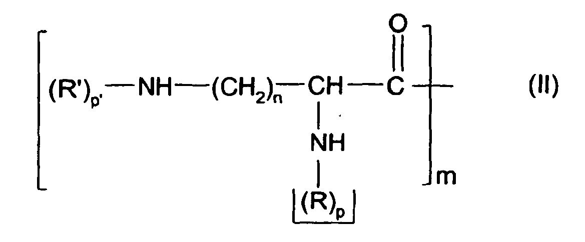
P différent de P' et P, P' = 0 ou 1
n = 3 ou 4
Quand
• P' = 0, le NH voisin est engagé dans une polymérisation N-ε; quand P = 0, le NH voisin est engagé dans une polymérisation N-α.
Quand
• P ou P' = 1, alors R ou R' = A'-SH
• A'= 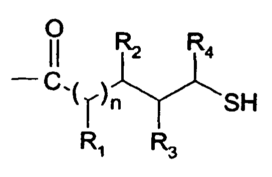
• R₁, R₂, R₃, R₄ = H, COOH, OH, NH₂, alkyl (C-1 à C-8) amino, acyl (C-1 à C-8) amino, acyl (C-1 à C-8) oxy, alkyl (C-1 à C-8) oxycarbonylamino, alkyl (C-1 à C-8) amino-carbonyloxy, halogéno, alkyl (C-1 à C-8) aminocarbonyl.
sachant que R ou R' peuvent, en partie seulement, également représenter
H, ou/et 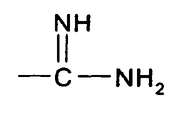 et ses sels ou/et 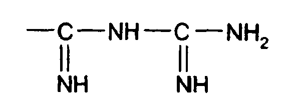 et ses sels.
• m = 3 à 10000.

12. Utilisation selon la revendication 11, **caractérisée par le fait que** le dérivé de poly- N- ε et poly N-α-lysine et ornithine à fonction thiol répondant à la formule II est appliqué sur la peau, les ongles ou les fibres kératiniques.

13. Procédé de préparation de dérivés de poly- N- ε et poly N-α-lysine et ornithine à fonction thiol de formule II ci-après: 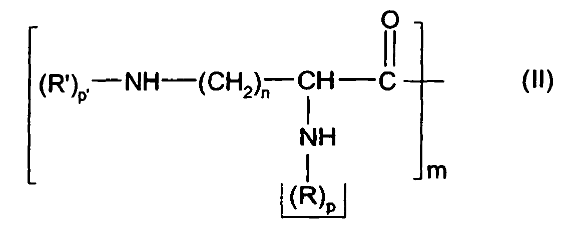
P différent de P' et P, P' = 0 ou 1
n = 3 ou 4
Quand
• P' = 0, le NH voisin est engagé dans une polymérisation N-ε; quand P = 0, le NH voisin est engagé dans une polymérisation N-α.
Quand
• P ou P' = 1, alors R ou R' = A'-SH
• A'= 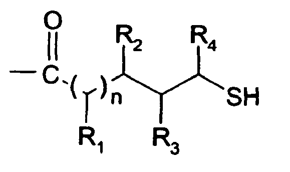
• R₁, R₂, R₃, R₄ = H, COOH, OH, NH₂, alkyl (C-1 à C-8) amino, acyl (C-1 à C-8) amino, acyl (C-1 à C-8) oxy, alkyl (C-1 à C-8) oxycarbonylamino, alkyl (C-1 à C-8) amino-carbonyloxy, halogéno, alkyl (C-1 à C-8) aminocarbonyl.
sachant que R ou R' peuvent, en partie seulement, également représenter
H, ou/et 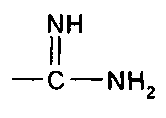 et ses sels ou/et 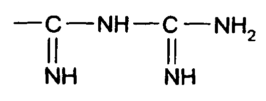 et ses sels.
• m = 3 à 10000,
par réaction, sous atmosphère inerte, de poly-N-ε-lysine ou de poly-N-ε-lysine à fonction guanidine ou biguanide avec une thiolactone.

## Claims

1. Use of a poly-N-α- and -N-ε-lysine and -ornithine of formula I, containing a thiol function, to form a remanant deposit on keratin materials, and especially on keratin fibres: 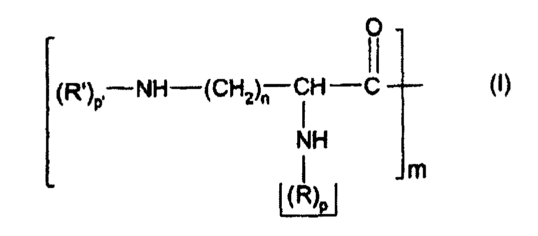 in which
P is different from P' and P, P' = 0 or 1;
n = 3 or 4;
if P' = 0, then the adjacent NH is engaged in an N-ε polymerization;
if P = 0, then the adjacent NH is engaged in an N-α polymerization;
if P or P' = 1, then R or R' = A-SH with
• A = saturated or unsaturated, linear or branched C₁ to C₃₀ hydrocarbon-based chain which may be interrupted with one or more hetero atoms or functions such as 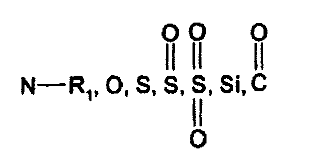 and/or with one or more aromatic or non-aromatic rings (5-, 6- or 7-membered rings) and which may be substituted with one or more groups: COOH, OH, NH₂, (C₁ to C₈) alkylamino, (C₁ to C₈) acylamino, (C₁ to C₈) acyloxy, (C₁ to C₈) alkyloxycarbonylamino, (C₁ to C₈) alkylaminocarbonyloxy, halo, (C₁ to C₈) alkylaminocarbonyl,
given that R or R' may, only in part, also represent
H and/or 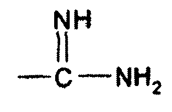 and salts thereof, and/or 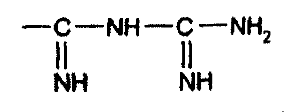 and salts thereof,
• R₁ = H, (C₁ to C₈) alkyl, (C₁ to C₈)acyl, (C₁ to C₈)alkyloxycarbonyl, (C₁ to C₈) alkylaminocarbonyl, halo,
• A may also be represented by a 5-, 6- or 7-membered aromatic or non-aromatic ring, optionally substituted with the same groups already listed for the aliphatic chain;
• m = 3 to 10 000.

2. Use according to Claim 1, the poly-N-α and -N-ε-lysine and -ornithine corresponding to formula I in which: 5 < m < 1000.

3. Use according to either one of the preceding claims, the poly-N-α and -N-ε-lysine and -ornithine having a degree of thiol-function grafting of greater than or equal to 1%.

4. Use according to any one of the preceding claims, the poly-N-α and -N-ε-lysine and -ornithine of formula I, containing a thiol function, being in combination with at least one conventional cosmetic active agent to form a remanant deposit on keratin materials.

5. Use according to Claim 4, the conventional cosmetic active agent being an anionic conventional cosmetic active agent, such as dyes, conditioners, moisturizers, emollients or sunscreens.

6. Derivatives of poly-N-ε- and poly-N-α-lysine and -ornithine, containing a thiol function, corresponding to formula II below: 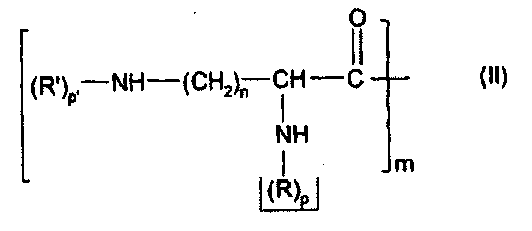
P is different from P' and P, P' = 0 or 1;
n = 3 or 4;
when
• P' = 0, the adjacent NH is engaged in an N-ε polymerization; when P = 0, the adjacent NH is engaged in an N-α, polymerization;
when
• P or P' = 1, then R or R' = A'-SH,
• A' = 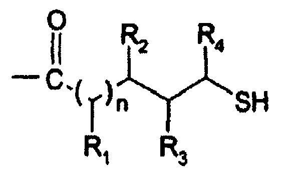
• R₁, R₂, R₃, R₄ = H, COOH, OH, NH₂, (C₁ to C₈) alkylamino, (C₁ to C₈) acylamino, (C₁ to C₈) acyloxy, (C₁ to C₈)alkyloxycarbonylamino, (C₁ to C₈) alkylaminocarbonyloxy, halo, (C₁ to C₈) alkylaminocarbonyl,
given that R or R' can, only in part, also represent
H, and/or 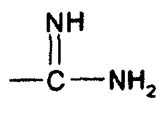 and salts thereof, and/or 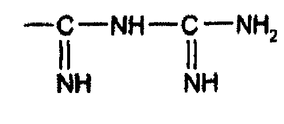 and salts thereof;
• m = 3 to 10 000.

7. Derivatives of poly-N-ε- and poly-N-α-lysine and -ornithine, containing a thiol function, according to Claim 6, **characterized in that** the degree of thiol-function grafting is greater than or equal to 1%.

8. Cosmetic composition comprising, in a cosmetically acceptable medium, at least one derivative of poly-N-ε- and poly-N-α-lysine and -ornithine, containing a thiol function, corresponding to formula II below: 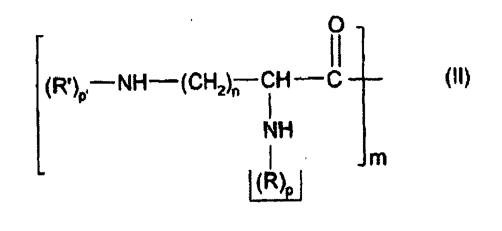
P is different from P' and P, P' = 0 or 1;
n = 3 or 4;
when
• P' = 0, the adjacent NH is engaged in an N-ε polymerization; when P = 0, the adjacent NH is engaged in an N-α polymerization;
when
• P or P' = 1, then R or R' = A'-SH,
• A'= 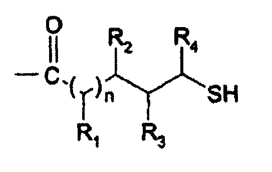
• R₁, R₂, R₃, R₄ = H, COOH, OH, NH₂, (C₁ to C₈)alkylamino, (C₁ to C₈) acylamino, (C₁ to C₈) acyloxy, (C₁ to C₈) alkyloxycarbonylamino, (C₁ to C₈) alkylaminocarbonyloxy, halo, (C₁ to C₈) alkylaminocarbonyl,
given that R or R' can, only in part, also represent
H, and/or 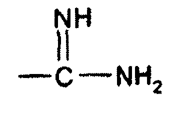 and salts thereof, and/or 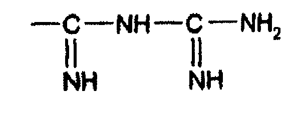 and salts thereof;
• m = 3 to 10 000.

9. Composition according to Claim 8, **characterized in that** the derivative(s) of poly-N-ε- and poly-N-α-lysine and -ornithine, containing a thiol function, of formula II is (are) present at concentrations of between 0.05% and 30% by weight, more preferably between 0.1% and 15% by weight, and more preferentially between 0.25% and 10% by weight, relative to the total weight of the composition.

10. Composition according to Claim 8 or 9, **characterized in that** the cosmetically acceptable medium is constituted of water and/or one or more cosmetically acceptable solvents such as alcohols, esters, ketones or cyclic volatile silicones or water-solvent(s) mixtures, these solvents preferably being C₁-C₄ alcohols.

11. Cosmetic use of derivatives of poly-N-ε- and poly-N-α-lysine and -ornithine, containing a thiol function, corresponding to formula II below: 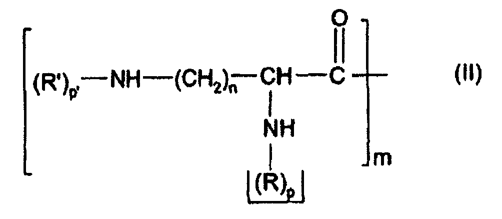
P is different from P' and P, P' = 0 or 1;
n = 3 or 4;
when
• P' = 0, the adjacent NH is engaged in an N-ε polymerization; when P = 0, the adjacent NH is engaged in an N-α polymerization;
when
• P or P' = 1, then R or R' = A'-SH,
• A'= 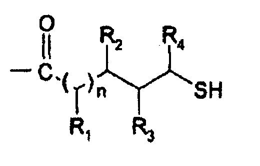
• R₁, R₂, R₃, R₄ = H, COOH, OH, NH₂, (C₁ to C₈) alkylamino, (C₁ to C₈)acylamino, (C₁ to C₈)acyloxy, (C₁ to C₈) alkyloxycarbonylamino, (C₁ to C₈)alkylaminocarbonyloxy, halo, (C₁ to C₈) alkylaminocarbonyl,
given that R or R' can, only in part, also represent
H, and/or 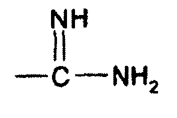 and salts thereof, and/or 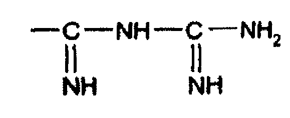 and salts thereof;
• m = 3 to 10 000.

12. Use according to Claim 11, **characterized in that** the derivative of poly-N-ε- and poly-N-α-lysine and -ornithine, containing a thiol function, corresponding to formula II is applied to the skin, the nails or keratin fibres.

13. Process for preparing derivatives of poly-N-ε- and poly-N-α-lysine and -ornithine, containing a thiol function, of formula II below: 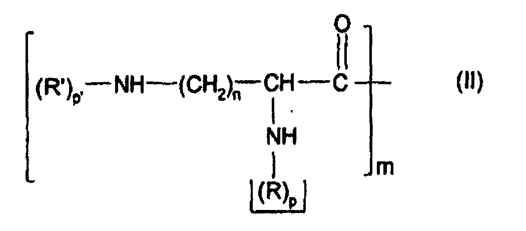
P is different from P' and P, P' = 0 or 1;
n = 3 or 4;
when
• P' = 0, the adjacent NH is engaged in an N-ε polymerization; when P = 0, the adjacent NH is engaged in an N-α polymerization;
when
• P or P' = 1, then R or R' = A'-SH,
• A'= 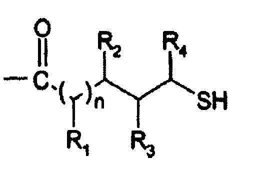
• R₁, R₂, R₃, R₄ = H, COOH, OH, NH₂, (C₁ to C₈)alkylamino, (C₁ to C₈) acylamino, (C₁ to C₈) acyloxy, (C₁ to C₈) alkyloxycarbonylamino, (C₁ to C₈) alkylaminocarbonyloxy, halo, (C₁ to C₈) alkylaminocarbonyl,
given that R or R' can, only in part, also represent
H, and/or 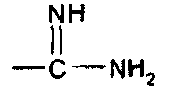 and salts thereof, and/or 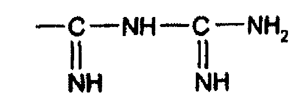 and salts thereof;
• m = 3 to 10 000,
by reacting poly-N-ε-lysine or poly-N-ε-lysine containing a guanidine or biguanide function with a thiolactone, under an inert atmosphere.

## Patentansprüche

1. Verwendung von Poly-N-α- und Poly-N-ε-lysin und -ornithin der Formel I mit Thiofunktion zur Bildung einer bleibenden Ablagerung auf Keratinsubstanzen und insbesondere Keratinfasern: 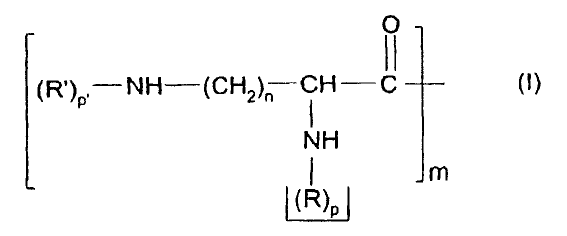 wobei in der Formel:
P ist von P' verschieden und P, P' = 0 oder 1;
n = 3 oder 4;
wenn P' = 0, ist das benachbarte NH bei einer N-ε-Polymerisation beteiligt;
wenn P = 0, ist das benachbarte NH bei einer N-α-Polymerisation beteiligt;
wenn P oder P' = 1, bedeuten R oder R' A-SH, mit
• A = eine gesättigte oder ungesättigte, geradkettige oder verzweigte Kohlenwasserstoffkette mit 1 bis 30 Kohlenstoffatomen, die unterbrochen sein kann durch ein oder mehrere Heteroatome oder eine oder mehrere Funktionen wie 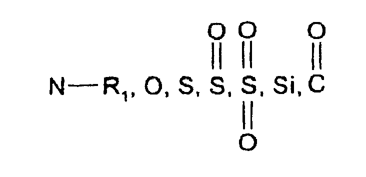 und/oder durch einen oder mehrere aromatische oder nichtaromatische Ringe (mit 5, 6 oder 7 Ringbestandteilen) und substituiert sein kann mit einer oder mehreren der folgenden Gruppen: COOH, OH, NH₂, Alkyl(C₁₋₈)amino, Acyl(C₁₋₈)amino, Acyl(C₁₋₈)oxy, Alkyl(C₁₋₈)oxycarbonylamino, Alkyl(C₁₋₈)amino-carbonyloxy, Halogen, Alkyl(C₁₋₈)aminocarbonyl,
wobei R oder R', nur zum Teil, auch bedeuten kann:
H und/oder 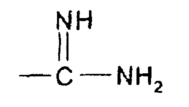 und seine Salze und/oder 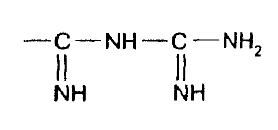 und seine Salze.
• R₁ = H, Alkyl(C₁₋₈), Acyl(C₁₋₈), Alkyl(C₁₋₈)oxycarbonyl, Alkyl(C₁₋₈)aminocarbonyl, Halogen
• A kann auch einen aromatischen oder nichtaromatischen, 5-, 6- oder 7-gliedrigen Ring bedeuten, dessen Ringbestandteile gegebenenfalls mit den oben bereits für die aliphatische Kette genannten Gruppen substituiert sein können
• m = 3 bis 10 000.

2. Verwendung nach Anspruch 1, wobei das Poly-N-α- und Poly-N-ε-lysin und -ornithin der Formel I entspricht, worin bedeuten: 5 < m < 1 000.

3. Verwendung nach einem der vorhergehenden Ansprüche, wobei das Poly-N-α- und Poly-N-ε-lysin und -ornithin einen Pfropfgrad der Thiofunktion von 1 % oder darüber aufweist.

4. Verwendung nach einem der vorhergehenden Ansprüche, wobei das Poly-N-α- und Poly-N-ε-lysin und -ornithin der Formel I mit Thiofunktion mit mindestens einem herkömmlichen kosmetischen Wirkstoff kombiniert wird, um auf Keratinsubstanzen eine bleibende Ablagerung zu bilden.

5. Verwendung nach Anspruch 4, wobei der herkömmliche kosmetische Wirkstoff ein herkömmlicher anionischer kosmetischer Wirkstoff ist, beispielsweise Farbmittel, Konditioniermittel, Hydratisierungsmittel, Emollientien oder Sonnenschutzfilter.

6. Derivate von Poly-N-ε- und Poly-N-α-lysin und -ornithin mit Thiofunktion der nachfolgenden Formel II: 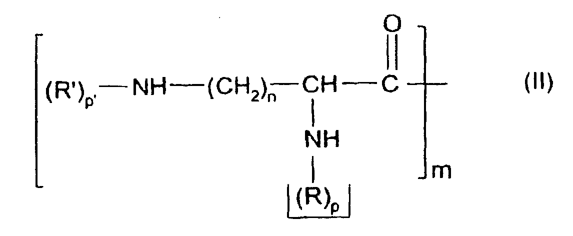 wobei P von P' verschieden ist und P, P' = 0 oder 1;
n = 3 oder 4
wenn:
• P' = 0, ist das benachbarte NH bei einer N-ε-Polymerisation beteiligt; wenn P = 0, ist das benachbarte NH bei einer N-α-Polymerisation beteiligt;
wenn:
• P oder P' = 1, dann bedeutet R oder R' = A'-SH
• A' = 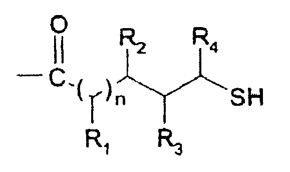
• R₁, R₂, R₃, R₄ = H, COOH, OH, NH₂, Alkyl(C₁₋₈)amino, Acyl(C₁₋₈)amino, Acyl(C₁₋₈)oxy, Alkyl(C₁₋₈)oxycarbonylamino, Alkyl(C₁₋₈)aminocarbonyloxy, Halogen, Alkyl(C₁₋₈)aminocarbonyl,
wobei R oder R', nur zum Teil, auch bedeuten können:
H und/oder 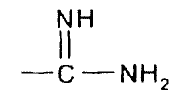 und seine Salze und/oder 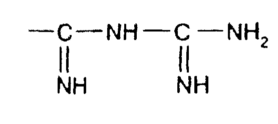 und seine Salze
• m = 3 bis 10 000.

7. Derivate von Poly-N-ε- und Poly-N-α-lysin und -ornithin mit Thiofunktion nach Anspruch 6, **dadurch gekennzeichnet, dass** der Pfropfungsgrad der Thiofunktion 1 % beträgt oder darüber liegt.

8. Kosmetische Zusammensetzung, die in einem kosmetisch akzeptablen Medium mindestens ein Poly-N-ε- und Poly-N-α-lysin-Derivat und -ornichin-Derivat mit Thiofunktion der Formel II enthält: 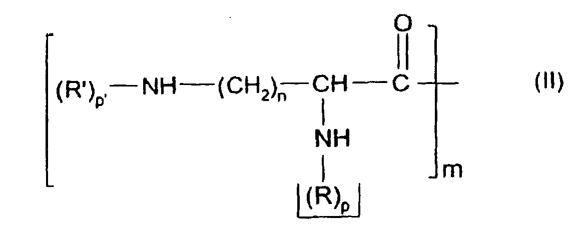 wobei P von P' verschieden ist und P, P' = 0 oder 1;
n = 3 oder 4;
wenn:
• P' = 0, ist das benachbarte NH bei einer N-ε-Polymerisation beteiligt; wenn P = 0, ist das benachbarte NH bei einer N-α-Polymerisation beteiligt
wenn:
• P oder P' = 1, dann bedeutet R oder R' = A'-SH;
• A' = 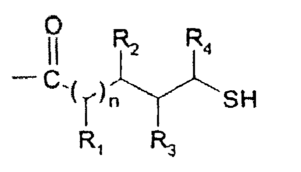
• R₁, R₂, R₃, R₄ = H, COOH, OH, NH₂, Alkyl(C₁₋₈)amino, Acyl(C₁₋₈)amino, Acyl(C₁₋₈)oxy, Alkyl(C₁₋₈)oxycarbonylamino, Alkyl(C₁₋₈)aminocarbonyloxy, Halogen, Alkyl(C₁₋₈)aminocarbonyl,
wobei R oder R', nur zum Teil, auch bedeuten können:
H und/oder 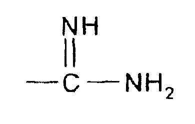 und seine Salze und/oder 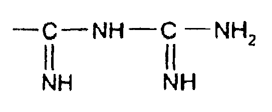 und seine Salze
• m = 3 bis 10 000.

9. Zusammensetzung nach Anspruch 8, **dadurch gekennzeichnet, dass** das oder die Derivat(e) von Poly-N-ε- und Poly-N-α-lysin und -ornithin mit Thiofunktion der Formel II in Konzentrationen von 0,05 bis 30 Gew.-%, vorzugsweise 0,1 bis 15 Gew.-% und noch bevorzugter 0,25 bis 10 Gew.-%, bezogen auf das Gesamtgewicht der Zusammensetzung, enthalten sind.

10. Zusammensetzung nach Anspruch 8 oder 9, **dadurch gekennzeichnet, dass** das kosmetisch akzeptable Medium aus Wasser und/oder einem oder mehreren kosmetisch akzeptablen Lösungsmitteln besteht, wie Alkoholen, Estern, Ketonen oder cyclischen flüchtigen Siliconen oder Gemischen Wasser-Lösungsmittel(n), wobei es sich bei den Lösungsmitteln vorzugsweise um C₁₋₄-Alkohole handelt.

11. Verwendung der Derivate von Poly-N-ε- und Poly-N-α-lysin und -ornithin mit Thiofunktion der folgenden Formel II in der Kosmetik: 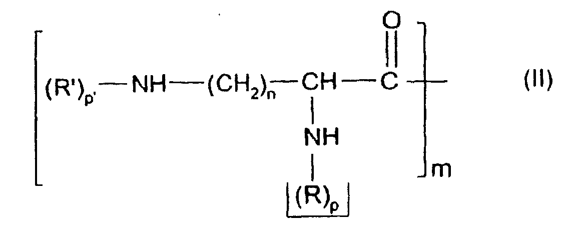 wobei P von P' verschieden ist und P, P' = 0 oder 1;
n = 3 oder 4;
wenn:
• P' = 0, ist das benachbarte NH bei einer N-ε-Polymerisation beteiligt; wenn P = 0, ist das benachbarte NH bei einer N-α-Polymerisation beteiligt;
wenn:
• P oder P' = 1, dann bedeutet R oder R' = A'-SH;
• A' = 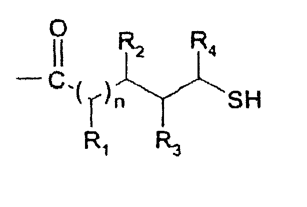
• R₁, R₂, R₃, R₄ = H, COOH, OH, NH₂, Alkyl(C₁₋₈)amino, Acyl(C₁₋₈)amino, Acyl(C₁₋₈)oxy, Alkyl(C₁₋₈)oxycarbonylamino, Alkyl(C₁₋₈)aminocarbonyloxy, Halogen, Alkyl(C₁₋₈)aminocarbonyl,
wobei R oder R', nur zum Teil, auch bedeuten können:
H und/oder 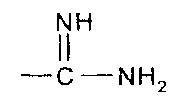 und seine Salze und/oder 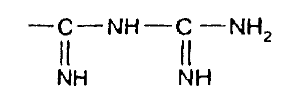 und seine Salze
• m = 3 bis 10 000.

12. Verwendung nach Anspruch 11, **dadurch gekennzeichnet, dass** das Poly-N-ε- und Poly-N-α-lysin-Derivat und -ornithin-Derivat mit Thiofunktion der Formel II auf die Haut, die Nägel oder die Keratinfasern aufgetragen wird.

13. Verfahren zur Herstellung von Derivaten von Poly-N-ε- und Poly-N-α-lysin und -ornithin mit Thiofunktion der folgenden Formel II: 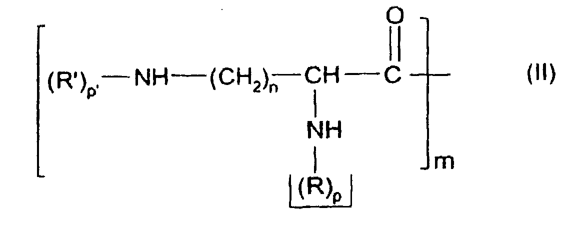 wobei P von P' verschieden ist und P, P' = 0 oder 1;
n = 3 oder 4;
wenn:
• P' = 0, ist das benachbarte NH bei einer N-ε-Polymerisation beteiligt; wenn P = 0, ist das benachbarte NH bei einer N-α-Polymerisation beteiligt
wenn:
• P oder P' = 1, dann bedeutet R oder R' = A'-SH;
• A' = 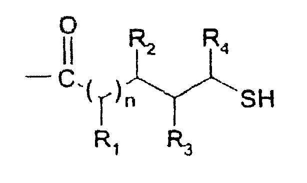
• R₁, R₂, R₃, R₄ = H, COOH, OH, NH₂, Alkyl(C₁₋₈)amino, Acyl(C₁₋₈)amino, Acyl(C₁₋₈)oxy, Alkyl(C₁₋₈)oxycarbonylamino, Alkyl(C₁₋₈)aminocarbonyloxy, Halogen, Alkyl(C₁₋₈)aminocarbonyl, wobei R oder R', nur zum Teil, auch bedeuten können:
H und/oder 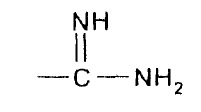 und seine Salze und/oder 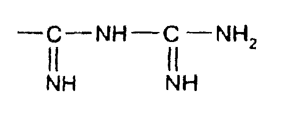 und seine Salze
• m = 3 bis 10 000,
durch Umsetzung von Poly-N-ε-lysin oder Poly-N-ε-lysin mit Guanidinfunktion oder Biguanidfunktion mit einem Thiolacton.
